# EUROPEAN PATENT SPECIFICATION

(11) **EP 1 833 543 B1**
(45) Date of publication and mention of the grant of the patent: **19.12.2012**
(21) Application number: 05817873.2
(22) Date of filing: 22.12.2005
(51) Int. Cl.: A61M 15/08

(54) **APPARATUS AND METHOD FOR FACILITATING THE TRANSMISSION OF VAPOUR PARTICLES DIRECTLY INTO A NASAL PASSAGE**
GERÄT UND VERFAHREN ZUR ERLEICHTERUNG DER ÜBERTRAGUNG VON DAMPFTEILCHEN DIREKT IN EINEN NASENGANG
APPAREIL ET PROCEDE POUR FACILITER LA TRANSMISSION DE PARTICULES DE VAPEUR DIRECTEMENT DANS LES VOIES NASALES

(30) Priority: 23.12.2004 SG 200407679
(43) Date of publication of application: 19.09.2007
(73) Proprietor: Tjia, Ng Ghee, Singapore 730702 (SG)
(72) Inventor: Tjia, Ng Ghee, Singapore 730702 (SG)
(74) Representative: Vogel, Andreas
(86) International application number: PCT/SG2005/000428
(87) International publication number: WO 2006/068624

(56) References cited:
- EP-A2- 1 214 956
- DE-U1- 20 319 549
- DE-U1- 20 319 549
- FR-A1- 2 845 920
- GB-A- 520 491
- GB-A- 768 488
- GB-A- 768 488
- US-A- 2 097 846
- US-A- 4 267 831
- US-A1- 2004 079 814

## Description

### FIELD OF INVENTION

The present invention reflates to an apparatus and method for facilitating the transmission of vapour particles directly into a nasal passage.

### BACKGROUND

Aromatherapy is the use of essences of plants processed as essential oils for therapeutic benefits such as, for example, attaining balance in a human body, enhancing the immunity system, preventing or minimizing illnesses, calming nerves, enhancing attention spans, and so forth. It is incorrect to generalise aromatherapy to be solely reliant on the sense of smell. This is because each essential oil has a unique combination of constituents which Interact with the body. It is this interaction with the constituents that provides the beneficial efforts for the body.

At the moment, common techniques used for aromatherapy are:
- Massaging of essential oil onto desired body part(s);
- Hot/Cold Compress: Application of a compress to the desired body part(s) with appropriate amounts of essential oils added to the compress maintained at a preferred temperature;
- Inhaling essential oils by vaporizing the oils using hot water in a basin or bowl;
- Inhaling essential oils by vaporizing the oils using aroma burners;
- Taking baths with drops of essential oils added to the water; and
- Direct inhalation from an absorbent material with drops of essential oils added.

it is widely acknowledged that direct inhalation of essential oils Is the best way of reaping the benefits of aromatherapy. Methods of vaporization mentioned above do not maximize the amount of essential oils being vaporized and, consequently, inhaled. There is significant wastage of essential oils when such methods are employed. As such, the amount of essential oils required must be increased, consequently increasing the cost incurred, Unfortunately, in order to make aromatherapy more economical, Impure essential oils augmented with synthetic Ingredients are used. Such practices are more often than not detrimental to health due to the inhalation of synthetic substances.

Furthermore, nasal inhalation provides more rapid effectiveness as compared to digestive, oral inhalation or dermal compress as the active ingredients are passed straight to the lungs where they are absorbed directly into the blood stream.

The Us patent 4, 267, 831 discloses an intranasal device forming an apparatus for transmission of vapour particles into a nasal passage. The apparatus comprises a structural member with holders at the ends of the structural member, each for insertion into the nasal passage. An absorbent member in each holder is thought to contain a liquid to be evaporated into the respiration air, which are performed in the form of an absorptive ring shaped washer made of porose material, providing the possibility of a clear nose respiration. The ring shape of the absorbent members comprising a central passage is necessary because the holders cover the entire nasal passage. The resilient containers would have the effect of a tight plug, when the central aperture in the absorptive washer would not be applied. Unfortunately, the washer shaped absorbent members are not optimized in evaporating the vapour particles into the respiration air.

The US Patent Application US 2004/0079814A1 discloses an intra nasal dispenser clip with a structural member and two absorbent members.

### SUMMARY

There Is provided an apparatus for facilitating the transmission of vapour particles directly into a nasal passage, including: at least one structural member, and at least one absorbent member at each end of the at least one structural member for containing a liquid able to be evaporated to form the vapour particles. Preferably, there are at least two studs positioned at intermediate positions between the ends of the at least one structural member.

Preferably, the at least one absorbent member is made from a material selected from: sponge, fabric, cotton, paper, wood, absorbent polymers and so forth. It is most preferable that the structural member is made from a material selected from: copper, aluminum, iron, steel, ductile polymers and so forth. The structural member may preferably be coated with a material selected from: PVC, silicon, latex, vinyl, nitrile, synthetic polymer, and so forth.

It is preferable that the at least one absorbent member Is located within a holder. The holder may preferably have a perforated disc in its inner rim. The holder may also have a solid disc in its Inner rim. The absorbent member may be in a shape selected from: disc-shaped, spherical-shaped, pyramid-shaped, polygonal-shaped, hemispherical- shaped, and so forth. The absorbent member may be used to contain liquid selected from: essential oils, or liquid medication. The liquid medication may be of the type typically introduced via nasal drops/sprays.

There Is also provided an apparatus for facilitating the transmission of vapour particles directly into a nasal passage, including: at least one structural member, and at least one absorbent member enveloping the at least one structural member for containing a liquid able to be evaporated to form the vapour particles.

There is also provided a method for the transmission of vapour partiles directly into a nasal passage using the aforementioned apparatus, and a method for the transmission of medication directly Into a nasal passage using the aforementioned apparatus.

### DESCRIPTION OF DRAVWNGS

In order that the invention may be better understood and readily put into practical effect, there shall now be described by way of non-limitative example only preferred embodiments of the present invention, the description being in reference to the accompanying illustrative drawings in which:
- Figure 1: is a perspective view of a preferred embodiment;
- Figure 2: is an exploded perspective view of a holder of a preferred embodiment;
- Figure 3: is top view of an absorbent member;
- Figure 4: is a perspective view of an alternative embodiment of the holder;
- Figure 5: is a perspective view of an alternative embodiment;
- Figure 6: Is a perspective view of another alternative embodiment in partial section;
- Figure 7: Is a perspective view of a further alternative embodiment;
- Figure 8: Is a side view of a preferred embodiment when In use; and
- Figure 9: is a view corresponding to Figure 2 of a final embodiment; and
- Figure 10: Is a partial side view of the embodiment of Figure 9 when assembled.

### DESCRIPTION OF PREFERRED EMBODIMENTS

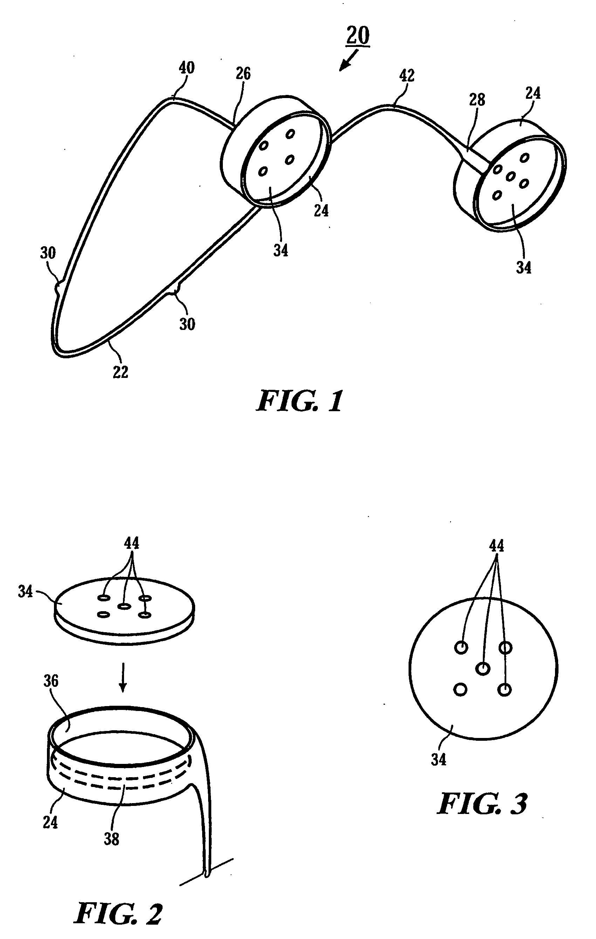

Referring to Figure 1, there is provided an apparatus 20 for facilitating the transmission of vapour particles directly Into a nasal passage. The apparatus 20 may include a structural member 22 with a holder 24 at each of a first end 26, and a second end 28, respectively, of the structural member 22. "At" in the case, may mean at, adjacent to, and In close proximity with. Absorbent members 34 may be located within each holder 24. The structural member 22 may have at least two studs 30 positioned at intermediate positions between the first 28 and second 28 ends of the structural member 22. The structural member 22 may be made of a plurality of components.

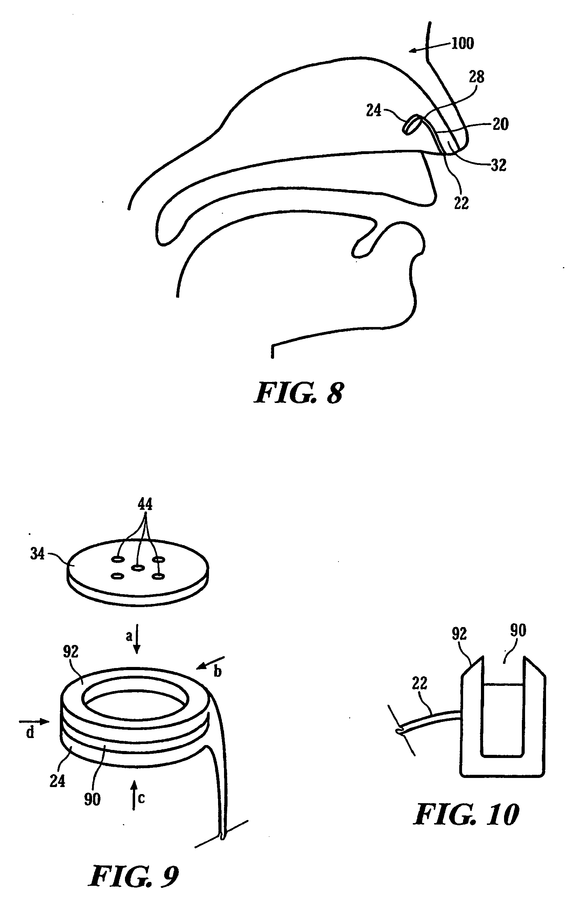

The structural member 22 may be flexible and bendable to form a U-shaped structure (as shown). The first 28 and second 28 ends of the structural member 22 may be bent from proximal positions 40 and 42 respectively. This may enhance the fit of the apparatus 20 In the nostrils of a user. The structural member 22 may be made from materials such as, for example, copper, aluminum, iron, steel, ductile polymers, and so forth. The structural member 22 may be coated with materials, such as, for example, PVC, silicon, latex, vinyl, nitrile, synthetic polymers, and so forth. The structural member 22 may be coated with the said materials as it may add comfort to a user of the apparatus 20 by being less abrasive, and by minimizing contact between the skin of the user (which may react adversely/ allergically to some materials) and the material of the structural member 22. The material used to coat the structural member 22 may also be skin coloured to camouflage the apparatus 20 when being used in the users' nostrils. The structural member 22 may be bent into a shape whereby the apparatus 20 may securely grip the central nasal bone. Figure 8 shows the apparatus 20 when In use In the nostrils 32 of a user 100.

The studs 30 positioned at Intermediate positions between the first 28 and second 28 ends of the structural member 22 may be located at positions that may enhance the grip-ability of the apparatus 20 for ease of handling.

An exploded perspective view of the holder 24 Is shown In Figure 2. The holder 24 may have a disc 38 fitted in the inner rim 38 of the holder 24. The disc 38 may be perforated to allow the passage of air. A disc-like absorbent member 34 is shown being placed into the holder 24. The absorbent member 34 may fit and conform to the inner rim 38 of the holder 24 and rest against the disc 38. The absorbent member 34 may be made from a liquid absorbing material such as, for example, sponge, fabric, cotton, paper, wood, absorbent polymers and so forth. The absorbent member 34 may be used to contain liquids, such as, for example, essential oils, or liquid medication. The liquid medication may be typically introduced via nasal drops/sprays. A solid disc may be preferred as it allows the absorbent member 34 to lose its contents through only one surface. This may slow the rate at which the absorbent member 34 dries (i.e. loses its liquid content). The holder 24 may prevent the liquid content in the absorbent member 34 from escaping from the absorbent member 34 as this may cause irritation/an allergic reaction to the user.

Figure 3 shows a top view of the absorbent member 34. The absorbent member 34 may have a plurality of through holes 44 in and through the absorbent member 34. The holes 44 may be present in the absorbent member 34 to facilitate the passage of air through the absorbent member 34 to aid in the evaporation of the liquid content in the absorbent member 34.

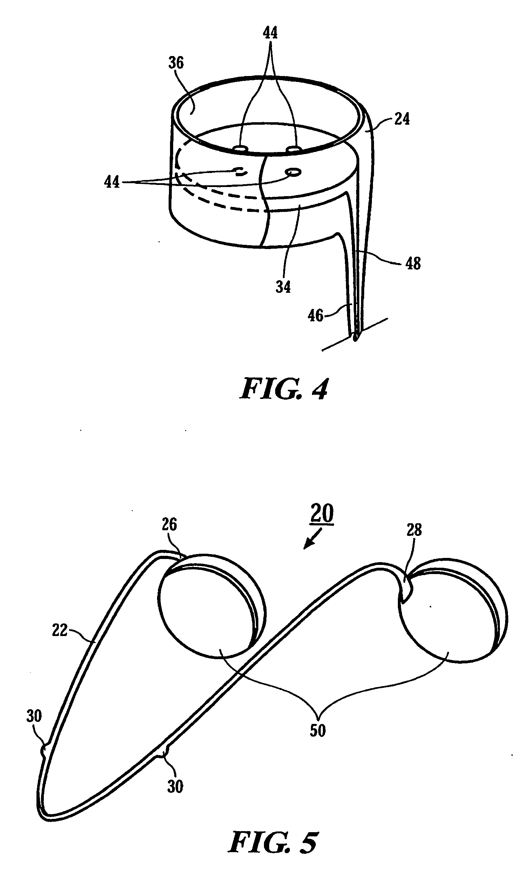

Figure 4 shows an alternative embodiment of the holder 24 of the present invention. The absorbent member 34 may be connected to a cavity 46 in the structural member 22. The cavity 46 may extend through all or part of the structural member 22 and acts as reservoir for the liquid. The cavity 46 may be used with a sponge 48 if desired or required. The holder 24 may also have a disc (not shown) located In the Inner rim 38 or the holder 24. The cavity 46 (with or without the sponge 48) increases the amount of liquid that may be stored for use by the absorbent member 34. The absorbent member 34 may also have through holes 44.

Figure 5 shows an alternative embodiment of the apparatus 20. The apparatus 20 may include a structural member 22 with absorbent members 50 located at a first end 26 and a second end 28 respectively of the structural member 22. The structural member 22 may be made from materials such as, for example, copper, aluminum, iron, steel, ductile polymers, and so forth. The structural member 22 may have at least two studs 30 positioned at Intermediate positions between the first 26 and second 28 ends of the structural member 22. The absorbent members 50 may be formed from materials, such as, for example, sponge, fabric, cotton, paper, absorbent polymers and so forth; and in different shapes, such as, for example, spherical-shaped, pyramid-shaped, polygonal-shaped, hemispherical-shaped, and so forth. The absorbent members 60 may be soaked with liquid content such as, for example, essential oils, and liquid medication. The liquid medication may be typically introduced via nasal drops/sprays. The apparatus 20 is used in an identical manner as the preferred embodiment of the present invention.

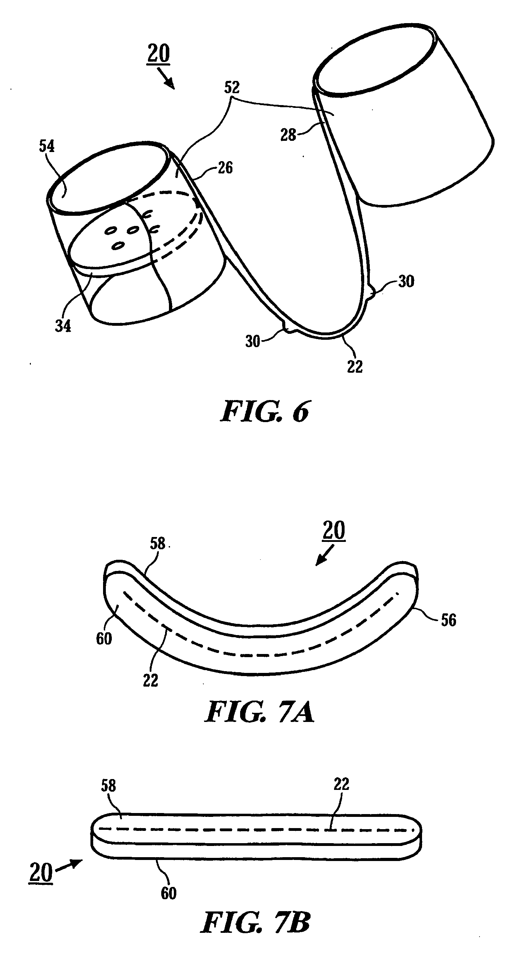

Referring to Figure 6, there is shown another alternative embodiment of the apparatus 20. The apparatus 20 may also include a structural member 22 The structural member 22 may be made from materials such as, for example, copper, aluminum, iron, steel, ductile polymers and so forth. The structural member 22 may have at least two studs 30 positioned at intermediate positions between the first 28 and second 28 ends of the structural member 22. The absorbent members 34 may be formed from materials, such as, for example, sponge, fabric, cotton, paper, absorbent polymers and so forth. The absorbent members 50 may be soaked with liquid content such as, for example, essential oils, and liquid medication. The liquid medication may be typically introduced via nasal drops/sprays. The apparatus 20 is used in an identical manner as the preferred embodiment of the present invention. However, this embodiment of the apparatus 20 may be less readily accepted as the users' would have the appearance of flared nostrils, and may be uncomfortable to the user.

Referring to Figure 7, there is shown yet another alternative embodiment of the apparatus 20. Figure 7a shows the apparatus 20 being bent prior to being placed into the nostrils of a user. Figure 7b shows the apparatus 20 prior to being bent. The apparatus 20 may have a structural member 22 enveloped by an absorbent member 56. The structural member 22 may be made from materials such as, for example, copper, aluminum, iron, steel, ductile polymers and so forth. The absorbent member 56 may be soaked with liquid content such as, for example, essential oils, and liquid medication, The liquid medication may be typically introduced via nasal drops/sprays. The absorbent member 56 may have an adhesive side 58 and a non-adhesive side 60. The adhesive side 58 may be used to adhere the apparatus 20 to the central nasal bone. The apparatus 20 may be bent into a U-shape with each end in each nostril with the adhesive side 58 adhering to the central nasal bone for anchorage to the central nasal bone. The non-adhesive side 80 may be exposed for the circulation of air on the absorbent member 56.

Figure 8 shows the apparatus 20 when in use. A second end 28 of the structural member 22 Is placed in one nostril 32 of a user 100 and the first end 26 of the structural member 22 is placed In the other nostril of the user 100. The structural member 22 may be bent by gripping studs at intermediate positions (not shown) between the first 28 and seconde 28 ends of the structural member 22. This allows for various sizes and shapes of noses, and for various sizes and separation of nostrils. The structural member 22 may be bent such that holder 24 does not cause discomfort to the user 100, and the apparatus 20 does not fall out from the nose of the user 100. Each holder 24 should have at least one absorbent member 34 soaked with liquid content such as, for example, essential oils, and liquid medication. The liquid medication may be typically introduced via nasal When the apparatus 20 is placed In such a position, the normal breathing of the user 100 would allow for the transmission of vapour particles into a nasal passage. The apparatus 20 may also be used to reduce the effect of foul smells in environments such as, for example, in washrooms, wet markets, vehicle exhaust emissions, smoking areas, sewage areas, rubbish clearing areas, and so forth. Due to the small size of the apparatus 20, coupled with the eliminating of the burning of essential oils, or external devices, it can be used anywhere and at any time. Given that the transmission of vapour particles is directly into the nasal passage of the user 100, discomfort is minimized to people surrounding the user 100 with sensitive noses, and who dislike the smell of the liquid content In the absorbent members 34.

Most essential oils are anti-septic to some degree. Hence, when the apparatus 20 is employed In the nostril 32 of the user 100, air may be cleansed before entering the nasal passage and, subsequently, the respiratory system.

Apparatus 20 may also be used to introduce medication through inhalation. There may be a way to use the apparatus 20 to aid smokers trying to quit smoking through the use of appropriate essential oils that may simulate the effects of nicotine or nicotine itself

Figures 9 and 10 show a variant of the embodiment of Figure 2. Here, the holder 24 has a top rim 92 through which the absorbent member 34 may pass. Each holder 24 also has an arcuate slot 90 extending around a part of the side wall of the holder 24, and through which the absorbent member 34 may also pass. There may be one or more slots 90 around the holder 24. Depending on the number and location of the slots, the absorbent member 34 may be passed into the holder 24 In the direction of one of the arrows a, b, c, or d.

Whilst there has been described in the foregoing description preferred embodiments of the present invention, It will be understood by those skilled in the technology concerned that many variations or modifications may be made to details of design or construction without departing from the present invention.

## Claims

1. An apparatus (20) for transmission of vapour particles directly into a nasal passage of a user, including:
a structural member (22);
a holder (24) at an end (26, 28) of the structural member (22) for insertion into the nasal passage;
an absorbent member (34) In the holder (24), the absorbent member (34) is for containing a liquid to be evaporated to form the vapour particles; and
a second holder (24) at a second end (26, 28) of the structural member (22) for insertion into the nasal passage;
a second absorbent member (34) in the second holder (24), the absorbent member (34) is for containing a liquid to be evaporated to form the vapor particles;
wherein each holder (24) is shaped and sized so as to allow passage of air between the holder (24) and the nasal passage,
**characterized in that**
each absorbent member (34) has a hole (44) for the passage of air through the hole (44) and allowing the direct transmission of the vapour particles Into the user's nasal passage.

2. The apparatus (20) of claim 1, wherein the structural member (22) includes a second end (28) and two studs (30) positioned at intermediate positions between the ends (26, 28) to grip a central nasal bone.

3. The apparatus (20) of claim 1, wherein the structural member (22) is a material selected from the group consisting of copper, aluminum, iron, steel and ductile polymers.

4. The apparatus (20) of claim 1, wherein the structural member (22) is coated with a material selected from the group consisting of PVC, silicon, latex, vinyl, nitrile and synthetic polymers.

5. The apparatus (20) of claim 1, wherein the holder (24) prevents the absorbent member (34) from contacting a nose that provides the nasal passage.

6. The apparatus (20) of claim 1, wherein the holder (24) prevents the liquid from escaping from the absorbent member (34).

7. The apparatus (20) of claim 1, wherein the holder (24) has an inner rim (36) that fits a disc (38), and the absorbent member (34) fits and conforms to the inner rim (36) and rests against the disc (38).

8. The apparatus (20) of claim 7, wherein the disc (38) is perforated for the passage of air therethrough within the nasal passage.

9. The apparatus (20) of claim 7, wherein the disc (38) is solid and allows the absorbent member (34) to lose its contents through only one surface.

10. The apparatus (20) of claim 1, wherein the holder (24) has a side wall with a slot (90) therein for the passage therethrough of the absorbent member (34).

11. The apparatus (20) of claim 1, wherein the absorbent member (34) contains the liquid.

12. The apparatus (20) of claim 1, wherein the absorbent member (34) has a plurality of holes (44) for the passage of air therethrough within the nasal passage.

13. The apparatus (20) of claim 1, wherein the absorbent member (34) includes opposing major surfaces perpendicular to the hole (44) and the hole (44) facilitates the passage of air therethrough within the nasal passage.

14. The apparatus (20) of claim 1, wherein the absorbent member (34) is shaped selected from the group consisting of disc-shaped, spherical-shaped, pyramid-shaped, polygonal-shaped, and hemispherical-shaped.

15. The apparatus (20) of claim 1, wherein the absorbent member (34) is a material selected from the group consisting of sponge, fabric, cotton, paper, wood, and absorbent polymers.

16. The apparatus (20) of claim 1, wherein the hole (44) is centrally located in the absorbent member (34).

17. The apparatus (20) of claim 1, wherein the hole (44) aids in the evaporation of the liquid from the absorbent member (34).

18. The apparatus (20) of claim 1, wherein the liquid provides aromatherapy for a user (100) with the nasal passage.

19. The apparatus (20) of claim 1, wherein the liquid provides medication for a user (100) with the nasal passage.

20. The apparatus (20) of claim 1, wherein the liquid is selected from the group consisting of essential oils, nicotine and liquid medication.

21. The apparatus (20) of claim 21, wherein the structural member (22) includes a cavity that extends through all or part of the structural member (22) and is in fluid communication with the absorbent members (34) and provides a reservoir for the liquid.

22. The apparatus (20) of claim 21, wherein the structural member (22) is bendable to form a U-shaped structure that grips a central nasal bone.

23. The apparatus (20) of claim 1, wherein the structural member (22) includes a cavity (46) that extends through all or part of the structural member (22) and is in fluid communication with the absorbent members (34) and provides a reservoir for the liquid.

24. An apparatus (20) of claim 1, wherein the holder (24) has an inner rim (36) for fitting therein of a disc (38).

## Patentansprüche

1. Apparatur (20) zur Übertragung von Dampfteilchen direkt in den Nasengang von einem Anwender, der Folgendes aufweist:
ein Strukturelement (22);
eine Halterung (24) an einem Ende (26, 28) von dem Strukturelement (22) zum Einsetzen in den Nasengang,
ein absorbierendes Element (34) in der Halterung (24), wobei das absorbierende Element (34) für die Aufnahme einer Flüssigkeit ist, die verdampft wird, um die Dampfteilchen zu bilden; und
eine zweite Halterung (24) an einem zweiten Ende (26, 28) von dem Strukturelement (22) zum Einsetzen in den Nasengang,
ein zweites absorbierendes Element (34) in der zweiten Halterung (24), wobei das absorbierende Element (34) für die Aufnahme einer Flüssigkeit ist, die verdampft wird, um die Dampfteilchen zu bilden; und
wobei jede Halterung (24) derart ausgeformt und dimensioniert ist, dass sie den Durchtritt von Luft zwischen der Halterung (24) und dem Nasengang gestattet.
**dadurch gekennzeichnet, dass** jedes absorbierende Element (34) ein Loch (44) für den Durchtritt von Luft durch das Loch (44) aufweist und die direkte Übertragung von den Dampfteilchen in den Nasengang des Anwenders gestattet.

2. Apparatur (20) nach Anspruch 1, wobei das Strukturelement (22) ein zweites Ende (28) und zwei Ansätze (30) aufweist, die an den Mittelpositionen zwischen den Enden (26, 28) positioniert sind, um das zentrale Nasenbein zu ergreifen.

3. Apparatur (20) nach Anspruch 1, wobei das Strukturelement (22) aus einem Material ist, das ausgewählt ist aus der Gruppe, die aus Kupfer, Aluminium, Eisen, Stahl und formbaren Polymeren besteht.

4. Apparatur (20) nach Anspruch 1, wobei das Strukturelement (22) mit einem Material beschichtet ist, das ausgewählt ist aus der Gruppe, die aus PVC, Silikon, Latex, Vinyl, Nitril und synthetischen Polymeren besteht.

5. Apparatur (20) nach Anspruch 1, wobei die Halterung (24) das Kontaktieren einer Nase, die den Nasengang vorsieht, durch das absorbierende Element (34) verhindert.

6. Apparatur (20) nach Anspruch 1, wobei die Halterung (24) das Entweichen der Flüssigkeit aus dem absorbierenden Element (34) verhindert.

7. Apparatur (20) nach Anspruch 1, wobei die Halterung (24) einen inneren Rand (36) aufweist, der eine Scheibe (38) einpasst, und das absorbierende Element (34) passt zu dem inneren Rand (36) und fügt sich diesem an und ruht auf de Scheibe (38).

8. Apparatur (20) nach Anspruch 7, wobei die Scheibe (38) für den Durchtritt von Luft dort hindurch innerhalb des Nasenganges durchlöchert ist.

9. Apparatur (20) nach Anspruch 7, wobei die Scheibe (38) massiv ist und es dem absorbierenden Element (34) gestattet, seinen Inhalt nur über eine Fläche abzugeben.

10. Apparatur (20) nach Anspruch 1, wobei die Halterung (24) eine Seitenwand mit einem Schlitz (90) darin für den Durchgang von dem absorbierenden Element (34) dort hindurch aufweist.

11. Apparatur (20) nach Anspruch 1, wobei das absorbierende Element (34) die Flüssigkeit enthält.

12. Apparatur (20) nach Anspruch 1, wobei das absorbierende Element (34) eine Vielzahl von Löchern (44) für den Durchtritt von Luft dort hindurch innerhalb des Nasenganges aufweist.

13. Apparatur (20) nach Anspruch 1, wobei das absorbierende Element (34) gegenüberliegende Hauptflächen enthält, die senkrecht zu dem Loch (44) sind, und das Loch (44) den Durchtritt von Luft dort hindurch innerhalb des Nasenganges ermöglicht.

14. Apparatur (20) nach Anspruch 1, wobei das absorbierende Element (34) eine Form aufweist, die ausgewählt ist aus der Gruppe, die aus einer scheibenförmigen, kugelförmigen, pyramidenförmigen, polygonal geformten und halbkugelförmigen Form besteht.

15. Apparatur (20) nach Anspruch 1, wobei das absorbierende Element (34) aus einem Material ist, das ausgewählt ist aus der Gruppe, die aus Schaumstoff, Stoff, Baumwolle, Papier, Holz und absorbierenden Polymeren besteht.

16. Apparatur (20) nach Anspruch 1, wobei das Loch (44) zentral in dem absorbierenden Element (34) platziert ist.

17. Apparatur (20) nach Anspruch 1, wobei das Loch (44) bei der Verdampfung der Flüssigkeit aus dem absorbierenden Element (34) unterstützt.

18. Apparatur (20) nach Anspruch 1, wobei die Flüssigkeit eine Aromatherapie für einen Anwender (100) über den Nasengang bereitstellt.

19. Apparatur (20) nach Anspruch 1, wobei die Flüssigkeit eine medikamentöse Behandlung für einen Anwender (100) über den Nasengang bereitstellt.

20. Apparatur (20) nach Anspruch 1, wobei die Flüssigkeit ausgewählt ist aus der Gruppe, die aus ätherischen Ölen, Nikotin und flüssigen Medikamenten besteht.

21. Apparatur (20) nach Anspruch 1, wobei das Strukturelement (22) einen Hohlraum aufweist, der sich durch das gesamte oder einen Abschnitt des Strukturelements (22) erstreckt und der in Fluidverbindung mit den absorbierenden Elementen (34) steht und einen Vorratsbehälter für die Flüssigkeit vorsieht.

22. Apparatur (20) nach Anspruch 1, wobei das Strukturelement (22) biegbar ist, um eine U-förmige Struktur zu bilden, die das zentrale Nasenbein ergreift.

23. Apparatur (20) nach Anspruch 1, wobei das Strukturelement (22) einen Hohlraum (46) aufweist, der sich durch das gesamte oder einen Abschnitt des Strukturelements (22) erstreckt und der in Fluidverbindung mit den absorbierenden Elementen (34) steht und einen Vorratsbehälter für die Flüssigkeit vorsieht.

24. Apparatur (20) nach Anspruch 1, wobei die Halterung (24) einen inneren Rand (36) aufweist, um darin eine Scheibe (38) einzupassen.

## Revendications

1. Appareil (20) de transmission de particules de vapeur directement dans les voies nasales d'un utilisateur, comprenant :
un élément structurel (22) ;
un support (24) à une extrémité (26, 28) de l'élément structurel (22), s'insérant dans les voies nasales,
un élément absorbant (34) dans le support (24), l'élément absorbant (34) étant destiné à contenir un liquide s'évaporant pour former les particules de vapeur ;
un second support (24) à une seconde extrémité (26, 28) de l'élément structurel (22), s'insérant dans les voies nasales,
un second élément absorbant (34) dans le second support (24), l'élément absorbant (34) étant destiné à contenir un liquide s'évaporant pour former les particules de vapeur,
chaque support (24) étant conformé et dimensionné de manière à permettre le passage d'air entre le support (24) et les voies nasales,
**caractérisé en ce que** cet élément absorbant (34) comporte
un trou (44) pour permettre le passage d'air dans le trou (44) et la transmission directe des particules de vapeur dans les voies nasales de l'utilisateur.

2. Appareil (20) selon la revendication 1, l'élément structurel (22) incluant une seconde extrémité (28) et deux clous (30) positionnés à des positions intermédiaires entre les extrémités (26, 28) pour saisir un os nasal central.

3. Appareil (20) selon la revendication 1, l'élément structurel (22) étant un matériau sélectionné dans le groupe composé du cuivre, de l'aluminium, du fer, de l'acier et des polymères ductiles.

4. Appareil (20) selon la revendication 1, l'élément structurel (22) étant revêtu d'un matériau sélectionné dans le groupe composé du PVC, du silicone, du latex, du vinyle, du nitrile et des polymères synthétiques.

5. Appareil (20) selon la revendication 1, le support (24) empêchant l'élément absorbant (34) de toucher un nez qui présente les voies nasales.

6. Appareil (20) selon la revendication 1, le support (24) empêchant le liquide de s'échapper de l'élément absorbant (34).

7. Appareil (20) selon la revendication 1, le support (24) présentant un bord interne (36) qui s'ajuste à un disque (38) et l'élément absorbant (34) s'adaptant et se conformant au bord interne (36) et reposant contre le disque (38).

8. Appareil (20) selon la revendication 7, le disque (38) étant perforé pour permettre le passage de l'air à travers lui dans les voies nasales.

9. Appareil (20) selon la revendication 7, le disque (38) étant solide et permettant à l'élément absorbant (34) de perdre son contenu seulement par une surface.

10. Appareil (20) selon la revendication 1, le support (24) présentant une paroi latérale dotée d'une fente (90) pour que l'élément absorbant (34) puisse passer à travers.

11. Appareil (20) selon la revendication 1, l'élément absorbant (34) contenant le liquide.

12. Appareil (20) selon la revendication 1, l'élément absorbant (34) présentant une pluralité de trous (44) pour permettre le passage d'air à travers lui dans les voies nasales.

13. Appareil (20) selon la revendication 1, l'élément absorbant (34) présentant des surfaces majeures opposées perpendiculaires au trou (44) et le trou (44) facilitant le passage d'air à travers lui dans les voies nasales.

14. Appareil (20) selon la revendication 1, l'élément absorbant (34) étant conformé en le sélectionnant dans le groupe composé comme suit : en forme de disque, en forme de sphère, en forme de pyramide, en forme de polygone et en forme d'hémisphère.

15. Appareil (20) selon la revendication 1, l'élément absorbant (34) étant un matériau sélectionné dans le groupe composé de l'éponge, du tissu, du coton, du papier, du bois et des polymères absorbants.

16. Appareil (20) selon la revendication 1, le trou (44) étant situé au centre de l'élément absorbant (34).

17. Appareil (20) selon la revendication 1, le trou (44) aidant à l'évaporation du liquide de l'élément absorbant (34).

18. Appareil (20) selon la revendication 1, le liquide fournissant de l'aromathérapie pour un utilisateur (100) par les voies nasales.

19. Appareil (20) selon la revendication 1, le liquide fournissant un médicament pour un utilisateur (100) par les voies nasales.

20. Appareil (20) selon la revendication 1, le liquide étant sélectionné dans le groupe composé des huiles essentielles, de la nicotine et des médicaments liquides.

21. Appareil (20) selon la revendication 1, l'élément structurel (22) comprenant une cavité qui s'étend à travers tout ou partie de l'élément structurel (22) et est en communication de fluide avec les éléments absorbants (34) et crée un réservoir pour le liquide.

22. Appareil (20) selon la revendication 1, l'élément structurel (22) pouvant être fléchi pour former une structure en forme de U qui saisit un os nasal central.

23. Appareil (20) selon la revendication 1, l'élément structurel (22) comprenant une cavité (46) qui s'étend à travers tout ou partie de l'élément structurel (22) et est en communication de fluide avec les éléments absorbants (34) et crée un réservoir pour le liquide.

24. Appareil (20) selon la revendication 1, le support (24) ayant un bord interne (36) permettant d'y adapter un disque (38).
